# EUROPEAN PATENT SPECIFICATION

(11) **EP 3 357 492 B1**
(45) Date of publication and mention of the grant of the patent: **04.12.2019**
(21) Application number: 18153151.8
(22) Date of filing: 24.01.2018
(51) Int. Cl.: A61K 9/24, A61K 33/06, A61K 33/08, A61K 33/10, A61P 3/02

(54) **MULTILAYER TABLET FOR ADMINISTRATION OF MAGNESIUM**
MEHRSCHICHTIGE TABLETTE FÜR VERABREICHUNG VON MAGNESIUM
COMPRIMÉ MULTICOUCHE POUR ADMINISTRATION DE MAGNÉSIUM

(30) Priority: 02.02.2017 IT 201700011337
(43) Date of publication of application: 08.08.2018
(73) Proprietor: S.I.I.T. S.r.l.-Servizio Internazionale Imballaggi Termosaldanti, 20090 Trezzano sul Naviglio MI (IT)
(72) Inventor: MARCELLONI, Luciano, 20090 Trezzano Sul Naviglio (MI) (IT); BENEGIAMO, Umberto, 20090 Trezzano Sul Naviglio (MI) (IT); MERICO, Roberto, 20090 Trezzano Sul Naviglio (MI) (IT)
(74) Representative: Bianchetti Bracco Minoja S.r.l.

(56) References cited:
- EP-A1- 1 661 575
- WO-A1-2011/078993
- US-A- 4 664 915
- US-A1- 2007 269 515
- US-A1- 2008 089 936

## Description

The present invention relates to a multilayer tablet for controlled release of magnesium, for use in the treatment of magnesium deficiency. The multilayer tablet according to the invention guarantees immediate release of magnesium into the stomach, and sustained release into the intestinal tract.

### Background to the invention

An important problem in the nutritional field relates to disorders or insufficiencies caused by magnesium deficiency which give rise to fatigue, a feeling of diffuse weakness, and irritability.

From the standpoint of supplementation, the administration of magnesium, especially magnesium salts, is very useful.

The greatest difficulty in obtaining sufficient blood levels of magnesium from forms of administration that cover a large part of the day is due to the poor absorption of magnesium salts.

The solubility of magnesium salts is greatest at acid pH values, whereas they tend to precipitate over certain pH values (starting at neutral pH values of around 7); moreover, in order to be absorbed effectively by the body, said magnesium salts must remain in solution.

Undissolved magnesium salts are not conveyed into the body through the intestinal villi, but excreted in the faeces. Examples of the most common magnesium-based salts and their physicochemical, solubility and pH characteristics are set out in the Table.

If magnesium release is to be sustained for several hours, it is essential to ensure absorption of said trace element in the intestine, where the gradually increasing pH constitutes a further complication, especially for solid oral forms such as tablets, which must provide the maximum blood levels of magnesium while only containing small amounts thereof compared with other oral forms, such as sachets with contents designed to be dissolved in water, or orodispersible sachets.

However, tablets are still the preferred form, due to the ease with which they can be taken by the user. EP 1 661 575 A1 discloses a two-layer tablet comprising (a) an immediate release layer comprising magnesium oxide, MCC, sodium croscarmellose and magnesium stearate, and (b) a delayed release layer comprising magnesium oxide, HPMC and magnesium stearate. The tablet is used to treat magnesium deficiency (more particularly, irritability, fatigue, etc).

To maximise the magnesium intake provided by a tablet, compounds wherein magnesium constitutes the largest percentage, such as magnesium oxide and hydroxide, are preferred.

However, the gradual increase in pH in the tract that runs from the duodenum to the small intestine in practice limits the dissolution of the magnesium released by a sustained-release system, because the biological fluids that penetrate said form are less effective in solubilising the magnesium residue.

This limitation particularly affects tablet forms, because the dissolution liquid is not always able to penetrate the form immediately.

**Table: common magnesium salts used in the nutritional field, and their properties**

| **Magnesium salt as** | **mg per 100 g salt** | **Solubility (g/100 g water)** | **pH (1% in water solution)** |
|---|---|---|---|
| Acetate 4 hydrate | 11 | 100 | 8 |
| Ascorbate 2 hydrate | 5.5 | 100 | 7 |
| Aspartate 4 hydrate | 6 | 5 | 7 |
| Carbonate | 25 | <1 | 10 |
| Chloride 6 hydrate | 12 | 310 | 5 |
| Citrate | 10 | 43 | 4 |
| Potassium citrate | 26 | 37 | 7.5 |
| Dicitrate | 15 | 24 | 9 |
| Fumarate | 11.5 | 12 | 7 |
| Gluconate | 5.5 | 19 | 7 |
| Glycerophosphate | 10/13 | 4 | 7 |
| Hydroxide | 41 | <1 | 10.5 |
| Lactate | 10 | 7 | 7 |
| Oxide | 58 | <1 | 10.5 |
| Phosphate 5 hydrate | 17.5 | <1 | 8 |
| Pidolate | 8.8 | 110 | 6 |
| Propionate | 13.5 | 1 | 10 |
| Phosphate | 15 | 40-50 | 6 |

### Description of the invention

It has now been found that the release of magnesium can be improved, and absorption in the gastrointestinal tract promoted, by the multilayer tablet for controlled release of a magnesium salt, oxide or hydroxide, for use in the treatment of states, disorders or conditions caused by magnesium deficiency, according to the present invention, which comprises:
(a) an immediate gastric release layer containing a magnesium oxide or hydroxide, or a magnesium salt selected from magnesium acetate, ascorbate, aspartate, carbonate, chloride, citrate, potassium citrate, dicitrate, fumarate, gluconate, glycerophosphate, lactate, phosphate 5 hydrate, pidolate, propionate and phosphate; a disintegrating agent and a binding polymer;
(b) a sustained-release layer containing (i) a polymer able to form a hydrogel matrix when it comes into contact with the fluids of the gastrointestinal tract, (ii) an organic acid able to dissociate in the gastrointestinal fluids, selected from citric acid, tartaric acid, ascorbic acid and lactic acid, and (iii) a magnesium oxide or hydroxide or a magnesium salt as defined above; and optionally,
(c) an inert layer inserted between layers (a) and (b), containing a disintegrating agent and a water-insoluble diluent, wherein said layers (a), (b) and (c) are exposed to the gastrointestinal fluids after the tablet is swallowed.

The amount of magnesium salt, oxide or hydroxide can range from 10 to 85% of the total tablet weight.

A disintegrating agent is needed to accelerate release from the immediate gastric layer. Disintegrating agents are materials that facilitate rapid disintegration of solid pharmaceutical forms by increasing the area in contact with the biological fluids. This can typically be effected by using materials that rapidly increase in volume or rapidly dissolve, creating channels in contact with water.

The disintegrating agent in the immediate-release layer according to the present invention is preferably selected from sodium carboxymethylcellulose, crosslinked polyvinylpyrrolidone (Kollidon® CL) and sodium starch glycolate (Explotab®). The agent sodium carboxymethylcellulose (Acdisol®) swells in contact with the gastric fluids, while crosslinked polyvinylpyrrolidone causes the layer to disintegrate within 30 minutes of contact due to rapid dissolution with the aqueous gastric fluids. The amount of disintegrating agent can range from 0.5% to 8%, preferably from 1% to 4%, of the total tablet weight.

The binding polymer has the function of increasing the cohesion of the powders, and is preferably selected from hydroxypropylcellulose, hydroxypropyl methylcellulose and polyvinylpyrrolidone. The amount of binding polymer present in the immediate-release layer can range from 1% to 5% of the total tablet weight.

In the sustained-release layer, the polymer able to form a hydrogel matrix when it comes into contact with the fluids of the gastrointestinal tract is preferably selected from medium- to high-molecular-weight hydroxypropyl methylcellulose, hydroxyethylcellulose, hydroxypropylcellulose and ethylcellulose. The amount of hydrogel matrix-forming polymer present in the sustained-release layer can range from 5% to 35% of the total tablet weight. In a preferred embodiment, the tablet will contain 45 mg to 135 mg, preferably 60 mg to 115 mg, of said polymer.

The organic acid dissociates in the gastrointestinal fluids and creates an acid microenvironment in the sustained-release layer, thus keeping the magnesium dissolved until it exits from the dosage form into the matrix diffusion layer at the interface with the external fluids, thereby increasing the magnesium levels absorbed into the blood. Said organic acid preferably has an acid dissociation constant pKa ranging between 3 and 7; it is selected from citric acid, tartaric acid, ascorbic acid and lactic acid. The amount of organic acid can range from 5 to 30% of the total tablet weight. The sustained-release layer preferably contains 50 mg to 250 mg of organic acid.

The inert layer between layers (a) and (b) prevents interaction between the gastric-release layer and the retard layer, and therefore also prevents alterations in their respective release kinetics. Said layer does not contain pharmacologically active ingredients, only excipients, in particular water-insoluble diluents, preferably selected from microcrystalline cellulose, corn or rice starch and calcium phosphate or carbonate, and disintegrating agents; it can also contain glidants.

The same excipients can be added to layers (a) and (b) to a variable extent, depending on technological and tablet-processing requirements. If not already present, all layers can also contain excipients and/or additives such as binders, disintegrating agents as defined above, lubricants that prevent the powders from adhering to mechanical parts during compression, sweeteners and flavourings.

The preparation process of the multilayer tablet according to the invention is that commonly employed for tablet preparation, and uses machines for granulation and drying of the ingredients, mixers and tablet presses, which are suitable for the preparation of multilayer tablets.

The mixture of ingredients can be prepared by the common techniques used to prepare mixtures intended to undergo direct compression or granulation of some of the ingredients (apart from bicarbonate, HPMC and polysaccharide), and subsequent mixing with powder lubricants and glidants.

The mixing stages are followed by compression in special rotary tablet presses able to generate tablets with multiple superimposed layers.

Said machines are typically fitted with at least two loading hoppers for the different mixtures corresponding to the different layers, and at least two compression stations, one after the other on the same rotor, where the two different mixtures to be compressed are batched.

In a typical preparation, a first hopper discharges the amounts of mixture of one of the three layers to be compressed, and the mixture is compressed in a first compression station. The half tablet obtained is then conveyed to the next compression station. There, it is coated with the second granulate batched by the second hopper, which is directly compressed onto the first layer, thus giving rise to the bilayer tablet.

The tablet according to the invention is for use in the treatment of states, disorders or conditions caused by magnesium deficiency, in particular those associated with fatigue, a sensation of weakness, and irritability.

The invention will be further illustrated by the examples below.

### Example 1 - 2-layer formula with tartaric acid as acidifier in the retard layer

| INGREDIENT | mg/tablet |
|---|---|
| Gastric-release layer | |
| Microcrystalline cellulose | 69.69 |
| Crosslinked polyvinylpyrrolidone | 45.00 |
| Magnesium oxide | 331.57 |
| Vegetable magnesium stearate | 6.50 |
| Colloidal silicon dioxide | 3.50 |
| Sodium carboxymethylcellulose | 10.00 |
| Red iron oxide (E172) | 1.50 |

| Retard Layer | |
|---|---|
| Magnesium hydroxide | 414.04 |
| Microcrystalline cellulose | 80.62 |
| Hydroxypropyl methylcellulose | 124.00 |
| Red iron oxide | 3.00 |
| Magnesium stearate | 6.00 |
| Colloidal silicon dioxide | 3.00 |
| Tartaric acid | 150.00 |

### Example 2 - 3-layer formula with tartaric acid as acidifier in the retard layer

| INGREDIENT | mg/tablet |
|---|---|
| Gastric-release layer | |
| Microcrystalline cellulose | 69.69 |
| Hydroxypropylcellulose | 45.00 |
| Magnesium oxide | 331.57 |
| Vegetable magnesium stearate | 6.50 |
| Colloidal silicon dioxide | 3.50 |
| Sodium carboxymethylcellulose | 10.00 |
| Red iron oxide (E172) | 1.50 |

| Inert layer | |
|---|---|
| Mannitol | 288.00 |
| Sodium carboxymethylcellulose | 4.50 |
| Vegetable magnesium stearate | 3.00 |
| Colloidal silicon dioxide | 4.50 |

| Retard Layer | |
|---|---|
| Magnesium hydroxide | 414.04 |
| Microcrystalline cellulose | 80.62 |
| Hydroxypropyl methylcellulose | 124.00 |
| Red iron oxide | 3.00 |
| Magnesium stearate | 6.00 |
| Colloidal silicon dioxide | 3.00 |
| Tartaric acid | 150.00 |

### Reference Example 3 - 3-layer comparison formula without acidifier in the retard layer

| INGREDIENT | mg/tablet |
|---|---|
| Gastric-release layer | |
| Microcrystalline cellulose | 69.69 |
| Hydroxypropylcellulose | 50.00 |
| Magnesium oxide | 331.57 |
| Vegetable magnesium stearate | 6.50 |
| Colloidal silicon dioxide | 3.50 |
| Sodium carboxymethylcellulose | 10.00 |
| Red iron oxide (E172) | 1.50 |

| Inert layer | |
|---|---|
| Mannitol | 288.00 |
| Sodium carboxymethylcellulose | 4.50 |
| Vegetable magnesium stearate | 3.00 |
| Colloidal silicon dioxide | 4.50 |

| Retard Layer | |
|---|---|
| Magnesium hydroxide | 414.04 |
| Microcrystalline cellulose | 80.62 |
| Hydroxypropyl methylcellulose | 124.00 |
| (Red iron oxide) | 3.00 |
| Magnesium stearate | 6.00 |
| Colloidal silicon dioxide | 3.00 |

### Example 4 - 3-layer formula with citric acid as acidifier in the retard layer

| INGREDIENT | mg/tablet |
|---|---|
| Gastric-release layer | |
| Microcrystalline cellulose | 69.69 |
| Hydroxypropylcellulose | 50.00 |
| Magnesium oxide | 331.57 |
| Vegetable magnesium stearate | 6.50 |
| Colloidal silicon dioxide | 3.50 |
| Red iron oxide (E172) | 1.50 |

| Inert layer | |
|---|---|
| Mannitol | 280.00 |
| Sodium carboxymethylcellulose | 4.50 |
| Vegetable magnesium stearate | 3.00 |
| Colloidal silicon dioxide | 4.50 |

| Retard Layer | |
|---|---|
| Magnesium hydroxide | 414.04 |
| Microcrystalline cellulose | 80.62 |
| Hydroxypropyl methylcellulose | 124.00 |
| Red iron oxide | 3.00 |
| Magnesium stearate | 6.00 |
| Colloidal silicon dioxide | 3.00 |
| Citric acid | 100.00 |

### Example 5

Same formula as example 4, with acidifier in the retard layer increased to 200 mg/tablet.

### Example 6 - In vitro study

To determine the amount of elemental magnesium released by the tablet, dissolution tests were conducted in water with added HCl and in water alone (without HCl), at the temperature of 37°C, with the paddle method at 100 rpm according to the European Pharmacopoeia monograph.
a) In 0.1N HCl, simulating the gastric residence time with the compositions described in reference example 3 and example 4.

### Results:

- tablet without acidifier:
   30 minutes: 183.47 mg/tab
   2 hours: 272.32 mg/tab
- tablet with acidifier:
   30 minutes: 191.21 mg/tab
   2 hours: 277.52 mg/tab.

As expected, complete dissolution of magnesium was observed in this test, but no substantial differences between the two formulations were observed because of the pH generated by the dissolution medium.

In order to study the real behaviour that takes place beyond the stomach, in a non-acid environment, the dissolution test was conducted in a medium consisting only of water:
b) the test was conducted in demineralised water alone, and differences in magnesium dissolution between the 2 formulations were observed:
- Batch PM423-4B-1C-10°: without addition of acidifier:
   2 hours: 4.82 mg/tab
   4 hours: 12.21 mg/tab
   8 hours: 16.60 mg/tab
- Batch PM423-5B-1C-10°: with addition of acidifier:
   2 hours: 8.77 mg/tab
   4 hours: 20.70 mg/tab
   8 hours: 28.11 mg/tab

The amount of magnesium dissolved is expected to increase in proportion to the amount of citric acid present (theoretically up to a maximum of 250 mg in the retard layer), for example by using the amounts of citric acid reported in example 5.

Finally, the larger amount of magnesium dissolved *in vitro,* starting with the form containing the acidifier in the retard layer, is expected to an even greater extent *in vivo,* because the dissolved magnesium is continually sequestered by the intestinal villi.

## Claims

1. A multilayer tablet for controlled release of a magnesium salt, oxide or hydroxide, for use in the treatment of states, disorders or conditions caused by magnesium deficiency, comprising:
(a) an immediate gastric release layer containing a magnesium oxide or hydroxide, or a magnesium salt selected from magnesium acetate, ascorbate, aspartate, carbonate, chloride, citrate, potassium citrate, dicitrate, fumarate, gluconate, glycerophosphate, lactate, phosphate 5 hydrate, pidolate, propionate and phosphate; a disintegrating agent and a binding polymer;
(b) a sustained-release layer containing (i) a polymer able to form a hydrogel matrix when it comes into contact with the fluids of the gastrointestinal tract, (ii) an organic acid able to dissociate in the gastrointestinal fluids, selected from citric acid, tartaric acid, ascorbic acid and lactic acid, and (iii) a magnesium oxide or hydroxide or a magnesium salt as defined above;
and optionally,
(c) an inert layer inserted between layers (a) and (b), containing a disintegrating agent and a water-insoluble diluent,
wherein said layers (a), (b) and (c) are exposed to the gastrointestinal fluids after the tablet is swallowed.

2. Tablet for use according to claim 1, wherein the amount of said magnesium salt, oxide or hydroxide ranges from 10 to 85% of the total weight of the tablet.

3. Tablet for use according to claim 1, wherein said disintegrating agent is selected from sodium carboxymethylcellulose, crosslinked polyvinylpyrrolidone and sodium starch glycolate.

4. Tablet for use according to claim 3, wherein said disintegrating agent is present in the immediate gastric release layer in amounts ranging from 0.5% to 8%, preferably from 1% to 4%, of the total weight of the tablet.

5. Tablet for use according to claim 1, wherein said binding polymer is selected from hydroxypropylcellulose, hydroxypropylmethylcellulose and polyvinylpyrrolidone.

6. Tablet for use according to claim 5, wherein said binding polymer is present in the immediate gastric release layer in amounts ranging from 1% to 5% of the total weight of the tablet.

7. Tablet for use according to claim 1, wherein said polymer which is able to form a hydrogel matrix when it comes into contact with the fluids of the gastrointestinal tract is selected from hydroxypropylmethylcellulose, hydroxypropylcellulose and hydroxyethylcellulose.

8. Tablet for use according to claim 7, wherein said polymer which is able to form a hydrogel matrix when it comes into contact with the fluids of the gastrointestinal tract is present in the sustained-release layer in amounts ranging from 5% to 35% of the total weight of the tablet.

9. Tablet for use according to claims 7-8, containing 45 mg to 135 mg, preferably 60 mg to 115 mg, of said polymer in the sustained-release layer.

10. Tablet for use according to claim 1, wherein said organic acid able to dissociate in the gastrointestinal fluids is present in the sustained-release layer in amounts ranging from 5 to 25%.

11. Tablet for use according to claim 1, wherein the water-insoluble diluent in the inert layer is selected from microcrystalline cellulose, corn or rice starch and calcium phosphate or carbonate.

12. Tablet for use according to the preceding claims, further comprising, in the layers wherein they are not already present, excipients and/or additives suitable for pharmaceutical use selected from binders, lubricants, glidants, sweeteners and natural flavourings.

13. Tablet for use according to claim 1, wherein said states, disorders or conditions are associated with fatigue, sense of weakness and irritability.

## Patentansprüche

1. Mehrschichttablette zur kontrollierten Freisetzung eines Magnesiumsalzes, -oxids oder -hydroxids zur Verwendung bei der Behandlung von Zuständen, Störungen oder Leiden, die durch Magnesiummangel verursacht werden, welche Folgendes umfasst:
(a) eine Schicht zur sofortigen Freisetzung im Magen, die ein Magnesiumoxid oder -hydroxid oder ein Magnesiumsalz ausgewählt aus Magnesiumacetat, - ascorbat, -aspartat, -carbonat, -chlorid, -citrat, -kaliumcitrat, -dicitrat, -fumarat, -gluconat, - glycerophosphat, -lactat, -phosphat-5-hydrat, - pidolat, -propionat und -phosphat, ein Zerfallsmittel und ein Bindungspolymer enthält;
(b) eine Schicht zur verzögerten Freisetzung, die (i) ein Polymer, das, wenn es mit den Fluiden des Magen-Darm-Traktes in Kontakt gelangt, zum Bilden einer Hydrogelmatrix in der Lage ist, (ii) eine organische Säure, die zum Dissoziieren in den Fluiden des Magen-Darm-Traktes in der Lage ist, ausgewählt aus Zitronensäure, Weinsäure, Ascorbinsäure und Milchsäure, und (iii) ein Magnesiumoxid oder -hydroxid oder ein Magnesiumsalz wie oben definiert enthält; und wahlweise
(c) eine inerte Schicht eingefügt zwischen Schicht (a) und (b), die ein Zerfallsmittel und ein wasserunlösliches Verdünnungsmittel enthält,
wobei die Schichten (a), (b) und (c) den Fluiden des Magen-Darm-Traktes ausgesetzt sind, nachdem die Tablette geschluckt wird.

2. Tablette zur Verwendung nach Anspruch 1, wobei die Menge des Magnesiumsalzes, -oxids oder -hydroxids im Bereich von 10 bis 85 % des Gesamtgewichts der Tablette liegt.

3. Tablette zur Verwendung nach Anspruch 1, wobei das Zerfallsmittel ausgewählt ist aus Natriumcarboxymethylcellulose, quervernetztem Polyvinylpyrrolidon und Natriumstärkeglycolat.

4. Tablette zur Verwendung nach Anspruch 3, wobei das Zerfallsmittel in der Schicht zur sofortigen Freisetzung im Magen in Mengen im Bereich von 0,5 % bis 8 %, vorzugsweise von 1 % bis 4 % des Gesamtgewichts der Tablette vorliegt.

5. Tablette zur Verwendung nach Anspruch 1, wobei das Bindungspolymer ausgewählt ist aus Hydroxypropylcellulose, Hydroxypropylmethylcellulose und Polyvinylpyrrolidon.

6. Tablette zur Verwendung nach Anspruch 5, wobei das Bindungspolymer in der Schicht zur sofortigen Freisetzung im Magen in Mengen im Bereich von 1 % bis 5 % des Gesamtgewichts der Tablette vorliegt.

7. Tablette zur Verwendung nach Anspruch 1, wobei das Polymer, welches, wenn es mit den Fluiden des Magen-Darm-Traktes in Kontakt gelangt, zum Bilden einer Hydrogelmatrix in der Lage ist, ausgewählt ist aus Hydroxypropylmethylcellulose, Hydroxypropylcellulose und Hydroxyethylcellulose.

8. Tablette zur Verwendung nach Anspruch 7, wobei das Polymer, welches, wenn es mit den Fluiden des Magen-Darm-Traktes in Kontakt gelangt, zum Bilden einer Hydrogelmatrix in der Lage ist, in der Schicht zur verzögerten Freisetzung in Mengen im Bereich von 5 % bis 35 % des Gesamtgewichts der Tablette vorliegt.

9. Tablette zur Verwendung nach Anspruch 7-8, welche 45 mg bis 135 mg, vorzugsweise 60 mg bis 115 mg des Polymers in der Schicht zur verzögerten Freisetzung enthält.

10. Tablette zur Verwendung nach Anspruch 1, wobei die organische Säure, die zum Dissoziieren in den Fluiden des Magen-Darm-Traktes in der Lage ist, in der Schicht zur verzögerten Freisetzung in Mengen im Bereich von 5 bis 25 % vorliegt.

11. Tablette zur Verwendung nach Anspruch 1, wobei das wasserunlösliche Verdünnungsmittel in der inerten Schicht ausgewählt ist aus mikrokristalliner Cellulose, Mais- oder Reisstärke und Calciumphosphat oder - carbonat.

12. Tablette zur Verwendung nach den vorhergehenden Ansprüchen, welche ferner in den Schichten, in welchen sie nicht bereits vorliegen, Hilfsstoffe und/oder Zusatzstoffe, die zur pharmazeutischen Verwendung geeignet sind, ausgewählt aus Bindemitteln, Schmiermitteln, Gleitstoffen, Süßungsmitteln und natürlichen Aromastoffen umfasst.

13. Tablette zur Verwendung nach Anspruch 1, wobei die Zustände, Störungen oder Leiden im Zusammenhang mit Ermüdung, Schwächegefühl und Reizbarkeit stehen.

## Revendications

1. Comprimé multicouche pour la libération contrôlée d'un sel, oxyde ou hydroxyde de magnésium, pour une utilisation dans le traitement d'états, troubles ou conditions dus à une insuffisance en magnésium, comprenant :
(a) une couche à libération gastrique immédiate contenant un oxyde ou hydroxyde de magnésium, ou un sel de magnésium choisi parmi l'acétate, l'ascorbate, l'aspartate, le carbonate, le chlorure, le citrate, le citrate potassique, le dicitrate, le fumarate, le gluconate, le glycérophosphate, le lactate, le phosphate 5-hydraté, le pidolate, le propionate et le phosphate de magnésium ; un agent délitant et un polymère liant ;
(b) une couche à libération prolongée contenant (i) un polymère capable de former une matrice d'hydrogel quand il vient au contact des fluides des voies gastro-intestinales, (ii) un acide organique capable de se dissocier dans les fluides gastro-intestinaux, choisi parmi l'acide citrique, l'acide tartrique, l'acide ascorbique et l'acide lactique, et (iii) un oxyde ou hydroxyde de magnésium ou un sel de magnésium tel que défini ci-dessus ;
et éventuellement (c) une couche inerte insérée entre les couches (a) et (b), contenant un agent délitant et un diluant insoluble dans l'eau,
dans lequel lesdites couches (a), (b) et (c) sont exposées aux fluides gastro-intestinaux après que le comprimé a été avalé.

2. Comprimé pour une utilisation selon la revendication 1, dans lequel la quantité dudit sel, oxyde ou hydroxyde de magnésium est située dans la plage allant de 10 à 85 % du poids total du comprimé.

3. Comprimé pour une utilisation selon la revendication 1, dans lequel ledit agent délitant est choisi parmi la carboxyméthylcellulose sodique ; la polyvinylpyrrolidone réticulée et le glycolate d'amidon sodique.

4. Comprimé pour une utilisation selon la revendication 3, dans lequel ledit agent délitant est présent dans la couche à libération gastrique immédiate en des quantités situées dans la plage allant de 0,5 % à 8 %, de préférence de 1 % à 4 %, du poids total du comprimé.

5. Comprimé pour une utilisation selon la revendication 1, dans lequel ledit polymère liant est choisi parmi l'hydroxypropylcellulose, l'hydroxypropylméthylcellulose et la polyvinylpyrrolidone.

6. Comprimé pour une utilisation selon la revendication 5, dans lequel ledit polymère liant est présent dans la couche à libération gastrique immédiate en des quantités situées dans la plage allant de 1 % à 5 % du poids total du comprimé.

7. Comprimé pour une utilisation selon la revendication 1, dans lequel ledit polymère qui est capable de former une matrice d'hydrogel quand il vient au contact des fluides des voies gastro-intestinales est choisi parmi l'hydroxypropylméthylcellulose, l'hydroxypropylcellulose et l'hydroxyéthylcellulose.

8. Comprimé pour une utilisation selon la revendication 7, dans lequel ledit polymère qui est capable de former une matrice d'hydrogel quand il vient au contact des fluides des voies gastro-intestinales est présent dans la couche à libération prolongée en des quantités situées dans la plage allant de 5 % à 35 % du poids total du comprimé.

9. Comprimé pour une utilisation selon les revendications 7 et 8, contenant 45 mg à 135 mg, de préférence 60 mg à 115 mg dudit polymère dans la couche à libération prolongée.

10. Comprimé pour une utilisation selon la revendication 1, dans lequel ledit acide organique capable de se dissocier dans les fluides gastro-intestinaux est présent dans la couche à libération prolongée en des quantités situées dans la plage allant de 5 à 25 %.

11. Comprimé pour une utilisation selon la revendication 1, dans lequel le diluant insoluble dans l'eau dans la couche inerte est choisi parmi la cellulose microcristalline, l'amidon de maïs ou de riz et le phosphate ou le carbonate de calcium.

12. Comprimé pour une utilisation selon les revendications précédentes, comprenant en outre, dans les couches où ils ne sont pas déjà présents, des excipients et/ou additifs convenant pour un usage pharmaceutique choisis parmi les liants, les lubrifiants, les agents glissants, les édulcorants et les arômes naturels.

13. Comprimé pour une utilisation selon la revendication 1, dans lequel lesdits états, troubles ou conditions sont associés à une fatigue, une sensation de faiblesse et une irritabilité.
